# EUROPÄISCHE PATENTSCHRIFT

(11) **EP 3 216 391 B1**
(45) Veröffentlichungstag und Bekanntmachung des Hinweises auf die Patenterteilung: **16.09.2020**
(21) Anmeldenummer: 17151768.3
(22) Anmeldetag: 17.01.2017
(51) Int. Cl.: A61B 5/02

(54) **VORRICHTUNG UND VERFAHREN ZUM ERMITTELN ZUMINDEST EINES INDIVIDUELLEN FLUIDDYNAMISCHEN KENNWERTS EINER STENOSE IN EINEM MEHRERE SERIELLE STENOSEN AUFWEISENDEN GEFÄSSSEGMENT**
DEVICE AND METHOD FOR DETERMINING AT LEAST ONE INDIVIDUAL FLUID DYNAMICS IDENTIFIER OF A STENOSIS IN A CONTAINER SEGMENT COMPRISING MULTIPLE STENOSES
DISPOSITIF ET PROCÉDÉ DE DÉTERMINATION D'AU MOINS UNE VALEUR CARACTÉRISTIQUE INDIVIDUELLE DE DYNAMIQUE DES FLUIDES D'UNE STÉNOSE DANS UN SEGMENT DE VAISSEAU PRÉSENTANT PLUSIEURS STÉNOSES EN SÉRIE

(30) Priorität: 09.03.2016 DE 102016203860
(43) Veröffentlichungstag der Anmeldung: 13.09.2017
(73) Patentinhaber: Siemens Healthcare GmbH, 91052 Erlangen (DE)
(72) Erfinder: Klingenbeck, Klaus, 91347 Aufseß (DE); Redel, Thomas, 91099 Poxdorf (DE); Scheuering, Michael, 90425 Nürnberg (DE); Wiets, Michael, 91094 Langensendelbach (DE)

(56) Entgegenhaltungen:
- US-A1- 2011 224 542
- US-A1- 2014 378 850
- US-A1- 2015 038 860
- US-A1- 2015 374 243
- US-A1- 2016 022 371

## Beschreibung

Die Erfindung betrifft ein Verfahren und eine Vorrichtung zum Ermitteln zumindest eines individuellen fluiddynamischen Kennwerts einer Stenose in einem mehrere serielle Stenosen aufweisenden Gefäßsegment. Als der fluiddynamische Kennwert kann beispielsweise die fraktionelle Flussreserve (FFR) ermittelt werden.

Eine Stenose stellt eine Verengung eines Körpergefäßes dar. Im Falle eines Blutgefäßes ist durch die Stenose der Blutfluss, das heißt die Hämodynamik, beeinträchtigt. Serielle Stenosen sind mehrere, stromaufwärts oder stromabwärts in dem Körpergefäß hintereinander angeordnete Stenosen. Möchte ein Arzt solche Stenosen behandeln, so wird bevorzugt mit der einflussreichsten oder signifikantesten Stenose begonnen. Beispielsweise kann mittels eines Stents das Blutgefäß im Bereich der Stenose gestützt oder geweitet werden. Heute ist die invasive FFR-Messung beispielsweise mittels eines Druckdrahtes der Standard in der Beurteilung der hämodynamischen Signifikanz von Stenosen. Liegen zwei oder mehrere serielle Stenosen in einem Gefäßabschnitt oder Gefäßsegment vor, kann zwar der integrale FFR-Wert, das heißt der Gesamtwert, invasiv gemessen werden, eine Messung der individuellen FFR-Werte jeder einzelnen Stenose ist jedoch nicht oder nur mit sehr hohem zusätzlichen Messaufwand möglich.

Eine für die klinische Entscheidungsfindung wichtige Frage im Falle mehrerer aufeinanderfolgender Stenosen ist aber diejenige nach dem individuellen FFR-Wert der einzelnen Stenosen.

Anstelle einer invasiven FFR-Messung kann auch eine sogenannte virtuelle FFR-Messung vorgenommen werden, welche nichtinvasiv einen FFR-Wert basierend auf Geometrieinformationen ermittelt, die zum Beispiel aus mehreren Angiographieaufnahmen gewonnen werden können. Hierzu ist aus dem Stand der Technik die Methode der CFD (computational fluid dynamics) bekannt.

Aus der US 2015/374243 A1 ist ein System zur Planung einer Operation zum Setzen von Stents in Stenosen koronarer Arterien bekannt, wobei auf der Grundlage von AngiographieAufnahmen einzelne Stenosen vermessen werden und daraufhin ein Wert betreffend den Druckabfall in jeder Stenose berechnet wird. Zum Berechnen des Druckabfalls ist als ein Eingabeparameter die Blutflussrate anzugeben. Falls mehrere Stenosen unmittelbar hintereinander angeordnet sind und für jede Stenose der Druckabfall ermittelt wird, beeinflussen sich die Stenosen gemäß dem Modell aufgrund dieser Blutflussrate gegenseitig.

Aus der US 2016/022371 A1 ist ein System bekannt, mittels welchem einem Arzt Vorschläge für die Behandlung von Stenosen mittels Stents gemacht werden. In Bezug auf den Einfluss einzelner Stents auf die Blutflussrate wird auf die US 2015/374243 A1 verwiesen.

Aus der US 2015/038860 A1 ist ein System zum patientenspezifischen Modellieren von Blutfluss bekannt, das auf Grundlage eines Modells für Blutgefäße einzelne Segmente der Blutgefäße ermittelt und diese durch Ersatzschaltungen nach bildet, um das Verhalten der Segmente in Bezug auf den Blutfluss zu berechnen. Das Modell kann ein 3D-Modell sein. Parameter der Ersatzschaltungen können mittels einer Simulation ermittelt werden. Alternativ dazu kann eine Zuordnung vorgesehen sein, die auf empirischen Modellen einer großen Population an patientenspezifischen Daten entwickelt sind.

Aus der US 2014/378850 A1 ist ein System zum Evaluieren eines Einflusses von Stenosen in Herzkranzgefäßen bekannt, wobei für einzelne Zweige koronarer Arterien Regionen mit möglichen Stenosen erkannt werden und dann durch Filtern und Verschmelzen solcher Regionen eine finale Stenose-Region ermittelt wird.

Der Erfindung liegt die Aufgabe zugrunde, bei einem Körpergefäß, das mehrere serielle Stenosen aufweist, einen individuellen fluiddynamischen Kennwert zumindest einer der Stenosen zu ermitteln.

Die Aufgabe wird durch die Gegenstände der unabhängigen Patentansprüche gelöst. Vorteilhafte Weiterbildungen der Erfindung sind durch die Merkmale der abhängigen Patentansprüche, die folgende Beschreibung sowie die Figuren offenbart.

Durch die Erfindung ist ein Verfahren zum Ermitteln zumindest eines individuellen fluidischen Kennwerts einer jeweiligen Stenose in einem mehrere serielle Stenosen aufweisenden Gefäßsegment eines Körpergefäßes bereitgestellt. Bei dem Körpergefäß kann es sich beispielsweise um ein Blutgefäß handeln. Das Verfahren sieht vor, dass aus einer Angiographie-Aufnahmeeinrichtung Angiographie-Bilddaten von dem Gefäßsegment empfangen werden. Bei der Aufnahmeeinrichtung kann es sich beispielsweise um ein Angiographiesystem oder ein CT-System (CT - Computer Tomographie) handeln. Durch eine Analyseeinrichtung werden auf der Grundlage der Angiographie-Bilddaten Geometriedaten des Gefäßsegments ermittelt und zu einem Segmentmodell des Gefäßsegments zusammengefasst. Dieses Segmentmodell beschreibt also die Geometrien oder Abmessungen des Gefäßsegments entlang seiner Längserstreckung stromabwärts oder stromaufwärts. Hierdurch sind auch die Stenosen, das heißt die Verengungen in dem Gefäßsegment abgebildet.

Um nun für die einzelnen Stenosen einen individuellen fluiddynamischen Kennwert ermitteln zu können, sieht das erfindungsgemäße Verfahren Folgendes vor. Durch eine Teilungseinrichtung wird in dem Segmentmodell zumindest eine Teilungsstelle mittels eines vorbestimmten Teilungskriteriums ermittelt. Jede Teilungsstelle befindet sich dabei jeweils zwischen zwei der Stenosen. Durch das Teilungskriterium wird festgelegt, welche Eigenschaft die Geometriedaten aufweisen müssen, damit die Teilungsstelle erkannt oder akzeptiert wird. Das Teilungskriterium gibt also geometrische Bedingungen vor, die vorliegen müssen, damit eine Stelle entlang des Segmentmodells als Teilungsstelle erkannt wird. Das Segmentmodell wird an der zumindest einen Teilungsstelle jeweils in Subsegmentmodelle unterteilt. Der an die Teilungsstelle stromaufwärts angrenzende Teil stellt ein Subsegmentmodell dar und der an die Teilungsstelle angrenzende stromabwärts liegende Teil stellt ein weiteres Subsegmentmodell dar. Zu beachten ist hierbei, dass das Segmentmodell dabei nicht verändert werden muss, da lediglich durch die Teilungsstelle festgelegt wird, wo der Übergang zwischen zwei Subsegmentmodellen angeordnet ist. Jedes Subsegmentmodell enthält zumindest eine der Stenosen. Mittels des Teilungskriteriums kann sichergestellt werden, dass bei der sich daraus ergebenden Einteilung des Segmentmodells jedes Subsegmentmodell eine einzelne Stenose enthält.

Durch eine Simulationseinrichtung wird dann für zumindest eines der Subsegmentmodelle auf der Grundlage jeweiliger Geometriedaten des Subsegmentmodells der jeweilige fluiddynamische Kennwert ermittelt. Durch die Teilungsstellen ist festgelegt, welche der Geometriedaten aus dem Segmentmodell als Geometriedaten des Subsegmentmodells für das Ermitteln des fluiddynamischen Kennwerts zugrundegelegt werden sollen. Die jeweiligen Geometriedaten jedes übrigen Subsegmentmodells werden ignoriert.

Jedes Subsegmentmodell erlaubt oder ermöglicht also eine individuelle Berechnung des fluiddynamischen Kennwerts, beispielsweise eines FFR-Werts, wobei nur Geometrieeigenschaften aus dem jeweiligen modellierten Subsegment verwendet werden. An den Trennungsstellen oder Teilungsstellen werden dabei Randbedingungen zugrundegelegt oder bereitgestellt, wie sie bei einem stenosefreien Gefäßverlauf jenseits der jeweiligen Teilungsstellen vorliegen würden.

Durch die Erfindung ergibt sich der Vorteil, dass das Berechnen des individuellen fluiddynamischen Kennwerts mit geringem Aufwand durchgeführt werden kann, da ausgehend von dem fertigen Segmentmodell für das gesamte Gefäßsegment nur die Randbedingungen an den Teilungsstellen ermittelt werden müssen, wie sie sich durch ein gesundes, das heißt stenosefreies, Gefäß ergeben würden. Das Segmentmodell selbst muss nicht angepasst werden. Stattdessen muss lediglich ein Ausschnitt des Segmentmodells verwendet werden, also derjenige Längenabschnitt, welcher dem Subsegment mit der darin enthaltenen Stenose entspricht und welcher durch die Teilungsstellen begrenzt oder identifiziert ist.

Gemäß der Erfindung wird das Segmentmodell an der Teilungsstelle nicht einfach abgeschnitten, sondern es kann das Subsegmentmodell an jeder Teilungsstelle z.B. durch Geometriedaten eines virtuellen, stenosefreien interpolierten Gefäßverlaufs ergänzt werden. Es kann also eine Interpolation des gesunden Gefäßes am Einlass und dem zumindest einen Auslass des jeweiligen Subsegmentmodells stattfinden.

Zu der Erfindung gehören auch optionale Weiterbildungen, durch deren Merkmale sich zusätzliche Vorteile ergeben.

Eine Weiterbildung sieht vor, dass das Segmentmodell das Gefäßsegment als ein 3D-Modell nachbildet. Dies kann beispielsweise im Falle von Angiographie-Bilddaten in Form von beispielsweise Röntgenprojektionen oder allgemeinen Projektionen, mittels einer Rückprojektion (back projection) gebildet sein. Das 3D-Modell kann z.B. auf sogenannten Voxels (Volume Elements - Volumenelementen) beruhen. Ein solches 3D-Modell weist den Vorteil auf, dass eine vollständige physikalische Simulation eines Fluidflusses, beispielsweise eines Blutflusses, durchgeführt werden kann.

Alternativ zu einem solchen 3D-Modell ist gemäß einer Weiterbildung vorgesehen, dass das Segmentmodell das Gefäßsegment als einen jeweiligen ortsabhängigen Verlauf eines Eigenschaftswerts zumindest einer Geometrieeigenschaft des Gefäßsegments beschreibt. Es wird also nicht das Gefäßsegment dreidimensional nachgebildet, sondern lediglich extrahierte Merkmale, nämlich die für die Kennwerte relevanten Geometrieeigenschaften, werden als Ortsfunktion aus den Angiographie-Bilddaten ermittelt oder extrahiert. Hierdurch ergibt sich der Vorteil, dass keine aufwändige Analyse eines dreidimensionalen Gebildes notwendig ist. Insbesondere kann hierdurch auch eine funktionale Zuordnung von Eigenschaftswerten zu einem fluiddynamischen Kennwert unmittelbar vorgenommen werden.

Bei der zumindest einen Geometrieeigenschaft handelt es sich insbesondere um einen Durchmesser, das heißt den Durchmesser über den Weg entlang des Gefäßsegments, und/oder eine durchströmbare Querschnittsfläche des Gefäßsegments. Diese Geometrieeigenschaften lassen sich zuverlässig aus Angiographie-Bilddaten extrahieren und bilden eine zuverlässige Grundlage zum Ermitteln eines fluiddynamischen Kennwerts.

Bevorzugt ist vorgesehen, dass das Segmentmodell zumindest einen Zustandswert eines physiologischen Körperzustands eines das Körpergefäß enthaltenden Körpers umfasst. Beispielsweise kann bei einem menschlichen Körper ein Pulsschlag und/oder ein Blutdruck und/oder ein Stresszustand und/oder eine Gefäßwandelastizität in vorteilhafter Weise durch diese Weiterbildung berücksichtigt werden.

Mehrere Weiterbildungen der Erfindung betreffen die Frage, wie das Segmentmodell in Subsegmente unterteilt werden kann. Eine Weiterbildung hierzu sieht vor, dass durch die Teilungseinrichtung die zumindest eine Teilungsstelle ermittelt wird, indem das hierfür zugrundegelegte Teilungskriterium umfasst, dass ein lokaler durchströmbarer Querschnitt des Gefäßsegments, also der Durchmesser und/oder die durchströmbare Querschnittsfläche, größer als ein vorbestimmter Prozentsatz des interpolierten, stenosefreien Gefäßverlaufs ist. Der Prozentsatz kann beispielsweise in einem Bereich von 50 Prozent bis 100 Prozent, bevorzugt in einem Bereich von 70 Prozent bis 100 Prozent, liegen. Hierdurch wird also ein stenosefreier oder durch Stenose unbeeinflusster Abschnitt definiert, wenn der durchströmbare Querschnitt lokal nur um 100 Prozent minus den besagten Prozentsatz von dem stenosefreien interpolierten Gefäßverlauf abweicht. Damit hierbei erkannt wird, dass man sich zwischen zwei Stenosen befindet, sieht das Teilungskriterium bevorzugt des Weiteren vor, dass stromaufwärts und/oder stromabwärts des besagten lokalen Querschnitts jeweils eine Stenose mit einem lokalen Querschnitt kleiner als der Prozentsatz angeordnet ist. Durch diese Weiterbildung wird es ermöglicht, dass das Segmentmodell automatisiert unterteilt werden kann.

Eine Weiterbildung sieht vor, dass einem Benutzer durch eine Anzeigeeinrichtung die Angiographie-Bilddaten und/oder das Segmentmodell angezeigt werden und als zumindest ein Teil des Teilungskriteriums eine manuelle Teilungsvorgabe von dem Benutzer empfangen wird. Diese Weiterbildung ermöglicht vorteilhafterweise einen korrigierenden oder hinweisenden Eingriff durch den Benutzer. Beispielsweise kann dem Benutzer auf einem Touchscreen (Berührungsbildschirm) das besagte 3D-Modell des Gefäßsegments angezeigt werden, sodass von dem Benutzer eine Berührung an dem Touchscreen empfangen werden kann und dies als Teilungsvorgabe für eine Teilungsstelle interpretiert werden kann.

Eine Weiterbildung sieht vor, dass eine Teilungsstelle zwischen zwei aneinander angrenzenden Subsegmentmodellen gelöscht wird, falls zumindest eines der beiden Subsegmentmodelle einen Längenabschnitt des Gefäßsegments beschreibt, der kleiner oder kürzer als eine vorbestimmte Mindestlänge ist. Mit anderen Worten umfasst das Teilungskriterium eine Vorgabe für eine Mindestlänge. Ist ein Subsegment des Gefäßsegments zu kurz, so werden Subsegmente zusammengefasst. Beispielsweise kann vorgesehen sein, dass ein Längenwert für eine Stent-Länge von dem Benutzer vorgegeben wird und dieser empfangene Längenwert als die Mindestlänge zugrundegelegt wird. Hierdurch ergibt sich der Vorteil, dass der Einfluss, den ein Stent auf das Gefäßsegment haben kann, unmittelbar berücksichtigt wird. Der Wert der Mindestlänge kann beispielsweise in einem Bereich von 5 Millimeter bis 2 Zentimeter liegen.

Eine Weiterbildung sieht vor, dass durch die Simulationseinrichtung der jeweilige Kennwert des zumindest einen Subsegmentmodells mittels einer Simulation eines Blutfluss in dem Subsegmentmodell ermittelt wird. Es wird also eine angiographische Kennwertermittlung mittels einer expliziten Modellierung des Blutflusses bereitgestellt. Hierdurch ergibt sich der Vorteil, dass wenige Modellannahmen beim Ermitteln des Kennwerts zugrundegelegt werden müssen. Dies ist insbesondere dann der Fall, wenn das besagte 3D-Modell verwendet wird.

Eine Weiterbildung sieht dagegen vor, dass durch die Simulationseinrichtung der jeweilige Kennwert des zumindest einen Submodells mittels einer Zuordnungsvorschrift zum Zuordnen der in dem Subsegmentmodell enthaltenen Geometriedaten zu dem Kennwert ermittelt wird. Es findet also eine implizite Ermittlung des Kennwerts in Abhängigkeit von den Geometriedaten des Subsegmentmodells statt, indem eine geeignete Zuordnungsvorschrift verwendet wird. Eine solche Zuordnungsvorschrift kann zusätzlich zu den Geometriedaten auch beispielsweise eine Angabe zum Fluidfluss, beispielsweise Blutfluss, zu Fluidparametern, beispielsweise Blutparametern, und/oder einer Gefäßelastizität des Gefäßsegments berücksichtigen oder umfassen.

Um eine geeignete Zuordnungsvorschrift zu finden, sieht eine Weiterbildung vor, dass die Zuordnungsvorschrift auf der Grundlage einer Methode des Maschinenlernens aus zumindest einer bekannten Zuordnung von Testgeometriedaten zu einem jeweiligen Kennwert gebildet wird. Mit anderen Worten wird aus bekannten fluiddynamischen Verhältnissen in einem Gefäßsegment, das die besagten Testgeometriedaten aufweist, und dem dazu bekannten fluiddynamischen Kennwert die Zuordnungsvorschrift automatisiert auf der Grundlage der Methode des Maschinenlernens ermittelt. Das Segmentmodell beschreibt hierbei bevorzugt nicht das 3D-Modell selbst, sondern den besagten ortsabhängigen Verlauf eines Eigenschaftswerts zumindest einer Geometrieeigenschaft des Gefäßsegments. Diese Weiterbildung weist den Vorteil auf, dass keine aufwändige dreidimensionale Modellierung nötig ist, sondern nur Merkmale oder Beschreibungen des Gefäßsegments, wie beispielsweise Länge und/oder Querschnitt, zugrundegelegt werden und diese dann einem fluiddynamischen Kennwert zugeordnet werden können.

Bevorzugt ist bei dem Verfahren vorgesehen, dass der fluiddynamische Kennwert ein Wert einer fraktionellen Flussreserve (FFR) ist, wie er im Zusammenhang mit der Auswertung von Stenosen als besonders geeignete Quantität anerkannt ist. Durch das Verfahren wird also bei dieser Weiterbildung für die Subsegmente oder Untersegmente eine individuelle FFR-Diagnostik bereitgestellt, wobei auf Grundlage des Segmentmodells für das gesamte Gefäßsegment und für individuelle Orte (Einlass und Auslass/Auslässe) mit geringem Aufwand der jeweilige FFR-Wert eines Subsegments ermittelt wird. Da das Ermitteln eines FFR-Werts nur hinter, das heißt stromabwärts einer jeweiligen Stenose aussagekräftig ist, wird durch das beschriebene Interpolieren mittels des virtuellen stenosefreien Gefäßverlaufs sichergestellt, dass sich aussagekräftige FFR-Werte ergeben.

Durch die lediglich teilweise Berücksichtigung der Geometriedaten, nämlich nur der Geometriedaten des jeweiligen Subsegmentmodells, kann es zu einem Offset oder Bias beim Berechnen eines fluiddynamischen Kennwerts kommen. Um diesen auszugleichen, gibt es eine vorteilhafte Weiterbildung der Erfindung. Diese Weiterbildung sieht vor, dass durch die Simulationseinrichtung mittels des gesamten Segmentmodells für das gesamte Gefäßsegment ein fluiddynamischer Gesamtkennwert ermittelt wird, welcher den Einfluss aller Stenosen beschreibt. Zusätzlich zu der Simulation für das gesamte Gefäßsegment sieht die Weiterbildung vor, dass Sensordaten einer Messeinrichtung, insbesondere eines Druckdrahtes, empfangen werden und mittels der Sensordaten und des Gesamtkennwerts der jeweilige ermittelte Kennwert des zumindest einen Subsegmentmodells kalibriert wird. Der einzelne fluiddynamische Kennwert und der Gesamtkennwert können hierbei gleichartige Kennwerte oder auch unterschiedliche Kennwerte sein. Der Gesamtkennwert kann beispielsweise den Blutfluss durch das Gefäßsegment angeben. Ein Druckdraht stellt einen Katheter dar, welcher einen drucksensitiven Sensor aufweist und welcher durch das Gefäßsegment bewegt werden kann, um hierbei Sensordaten betreffend einen ortsabhängigen Druckwert zu erzeugen. Mittels solcher Sensordaten kann beispielsweise eine Korrektur oder Kalibrierung des Kennwerts vorgenommen werden, falls beispielsweise der von dem Gefäßsegment geführte Blutfluss falsch eingeschätzt oder geschätzt worden ist.

Um das erfindungsgemäße Verfahren durchzuführen, ist durch die Erfindung eine entsprechende Vorrichtung bereitgestellt, die eine Empfangseinrichtung und eine Prozessoreinrichtung umfasst. Die Empfangseinrichtung ist dazu eingerichtet, von der besagten Angiographie-Aufnahmeeinrichtung die Angiographie-Bilddaten von dem Gefäßsegment zu empfangen. Die Prozessoreinrichtung umfasst die besagte Analyseeinrichtung zum Ermitteln eines Segmentmodells mit Geometriedaten des Gefäßsegments aus den Angiographie-Bilddaten, die besagte Teilungseinrichtung zum Ermitteln zumindest einer Teilungsstelle zwischen jeweils zwei der Stenosen in dem Segmentmodell mittels des besagten Teilungskriteriums und zum Einteilen des Segmentmodells in die Subsegmentmodelle anhand der zumindest einen Teilungsstelle sowie die besagte Simulationseinrichtung zum Ermitteln des jeweiligen fluiddynamischen Kennwerts für zumindest eines der Subsegmentmodelle auf der Grundlage der jeweiligen Geometriedaten des Subsegmentmodells. Die Vorrichtung ist dazu eingerichtet, eine Ausführungsform des erfindungsgemäßen Verfahrens durchzuführen. Mittels der Vorrichtung kann beispielsweise eine diagnostische Analyse für die individuellen Stenosen durchgeführt werden, damit ein Benutzer beispielsweise entscheiden kann, welche der Stenosen den Vorrang bei der Behandlung beispielsweise mittels Stents bekommen soll, weil sie den größten Einfluss auf die Fluiddynamik, z.B. die Hämodynamik, aufweist.

Im Folgenden ist ein Ausführungsbeispiel der Erfindung beschrieben. Hierzu zeigt:
- FIG 1: eine schematische Darstellung einer Ausführungsform der erfindungsgemäßen Vorrichtung;
- FIG 2: eine schematische Darstellung eines Segmentmodells eines Gefäßsegments, in welchem fluiddynamische Kennwerte für zumindest eine von mehreren seriellen Stenosen ermittelt werden sollen;
- FIG 3: eine schematische Darstellung eines weiteren Segmentmodells, das auf einem ortsabhängigen Verlauf eines Eigenschaftswerts einer Geometrieeigenschaft basiert;
- FIG 4: einen weiteren Teil des Segmentmodells von FIG 3, wobei ein weiterer ortsabhängiger Verlauf eines Eigenschaftsworts einer Geometrieeigenschaft beschrieben ist; und
- FIG 5: eine schematische Darstellung eines Segmentmodells, das an einer Teilungsstelle in zwei Subsegmentmodelle unterteilt ist.

Bei dem im Folgenden erläuterten Ausführungsbeispiel handelt es sich um eine bevorzugte Ausführungsform der Erfindung. Bei dem Ausführungsbeispiel stellen die beschriebenen Komponenten der Ausführungsform jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren ist die beschriebene Ausführungsform auch durch weitere der bereits beschriebenen Merkmale der Erfindung ergänzbar.

In den Figuren sind funktionsgleiche Elemente jeweils mit denselben Bezugszeichen versehen.

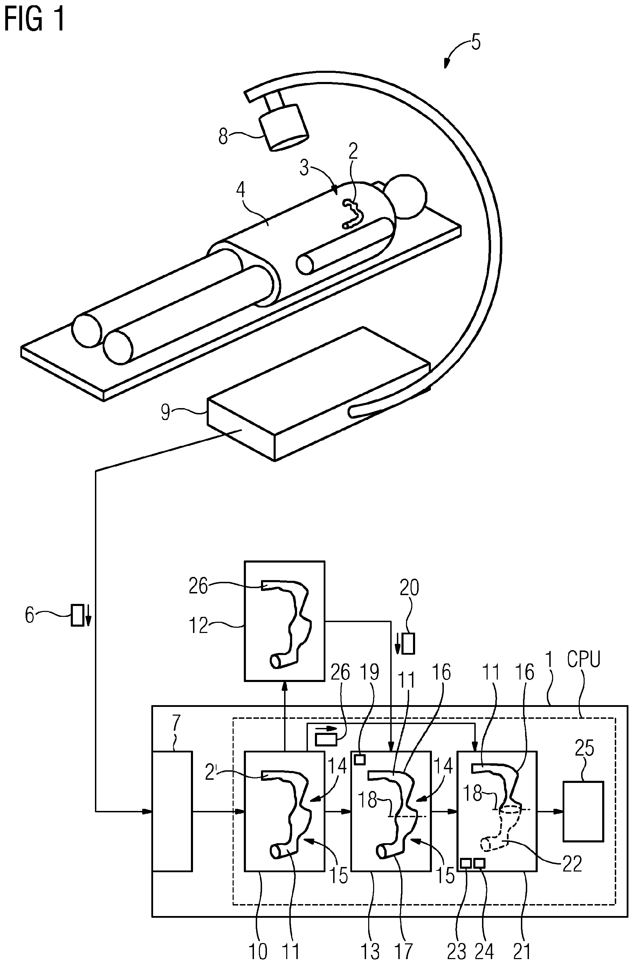

FIG 1 zeigt eine Vorrichtung 1, mittels welcher ein Gefäßsegment 2 eines Körpergefäßes 3 eines Körpers 4 beispielsweise eines Patienten untersucht werden kann. Die Vorrichtung 1 kann hierzu von einer Angiographie-Aufnahmeeinrichtung 5 Angiographie-Bilddaten 6 empfangen. Hierzu kann die Vorrichtung 1 eine Empfangseinrichtung 7 aufweisen, über welche die Vorrichtung 1 mit der Angiographie-Aufnahmeeinrichtung 5 gekoppelt sein kann. Die Angiographie-Bilddaten 6 können auch beispielsweise mittels eines Speichermediums an die Empfangseinrichtung 7 übertragen werden. Bei der Angiographie-Aufnahmeeinrichtung 5 kann es sich beispielsweise um ein Angiographiesystem oder einen Computertumorgraphen handeln. Zum Ermitteln der Angiographie-Bilddaten kann beispielsweise ein Kontrastmittel in das Körpergefäß 3 gespritzt und anschließend das Körpergefäß 3 mittels einer Röntgenquelle 8 bestrahlt werden, sodass das Körpergefäß 3 mit dem darin enthaltenen Kontrastmittel auf einem Röntgen-Detektor 9 abgebildet ist. Die pixelbasierten Detektordaten sind dann Bestandteil der Angiographie-Bilddaten 6. Es kann vorgesehen sein, das Körpergefäß 3 aus unterschiedlichen Portionswinkeln abzubilden.

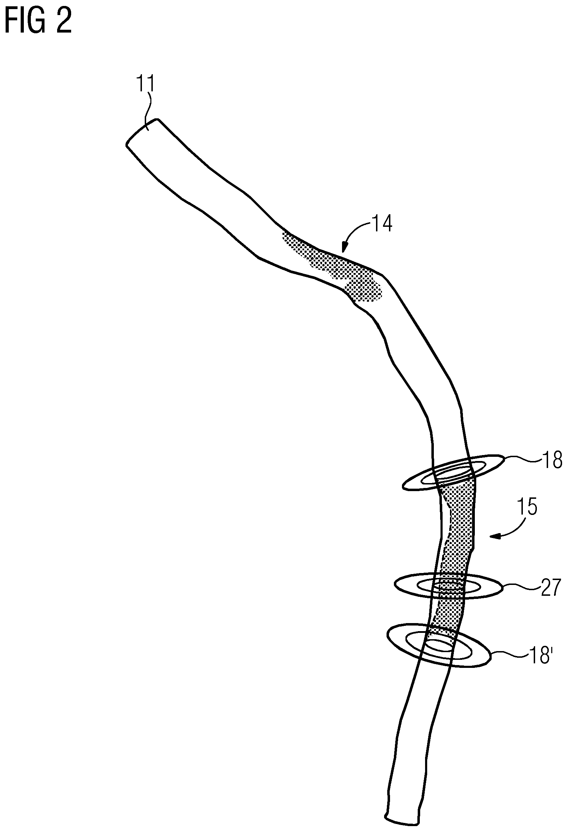

Die von der Empfangseinrichtung 7 empfangenen Angiographie-Bilddaten 6 können durch eine Analyseeinrichtung 10 empfangen werden. Die Analyseeinrichtung 10 kann die Angiographie-Bilddaten zu einem Segmentmodell 11 zusammenfassen, welches eine Beschreibung oder Abbildung 2' des Gefäßsegments 2 darstellt. Das Segmentmodell 11 kann durch die Vorrichtung 1 auf einer Anzeigeeinrichtung 12, beispielsweise einem Bildschirm, einem (nicht dargestellten) Benutzer der Vorrichtung 1 angezeigt werden.

Das Gefäßsegment 2 kann mehrere seriell hintereinander angeordnete Stenosen 14, 15 aufweisen, zu denen mittels der Vorrichtung 1 jeweils ein fluiddynamischer Kennwert, insbesondere ein FFR-Wert, ermittelt werden kann.

Eine Teilungseinrichtung 13 kann hierzu das Segmentmodell 11 unterteilen, sodass sich Subsegmentmodelle 16, 17 ergeben, von denen jedes jeweils nur eine der Stenosen 14, 15 umfasst. Zum Festlegen der Subsegmentmodelle 16, 17 wird durch die Teilungseinrichtung 13 jeweils eine Teilungsstelle 18 zwischen den Stenosen 14, 15 festgelegt. Hierzu wird ein vorbestimmtes Teilungskriterium 19 zugrundegelegt. Beispielsweise kann als ein Bestandteil des Teilungskriteriums 19 eine Benutzereingabe oder Teilungsvorgabe 20 von dem Benutzer empfangen werden. Hierzu kann beispielsweise an der Anzeigeeinrichtung 12, falls diese ein Touchscreen ist, eine entsprechende Berühreingabe an der Anzeigeeinrichtung 12 vom Benutzer empfangen werden. Eine automatisierte Festlegung der zumindest einen Teilungsstelle 18 wird im Zusammenhang mit den folgenden Figuren beschrieben.

Um nun für eines der Subsegmentmodelle 16 den fluiddynamischen Kennwert festzulegen, kann durch eine Simulationseinrichtung 21 von dem Segmentmodell 11 derjenige Teil oder Inhalt genutzt werden, welcher diejenigen Geometriedaten enthält, die zum Subsegmentmodell 16 gehören. Anstelle des verbleibenden Subsegmentmodells 17, das heißt des übrigen Teils des Segmentmodells 11, wird ein virtueller, stenosefreier interpolierter Gefäßverlauf 22 zugrundegelegt, dessen fluiddynamisches Verhalten an der Teilungsstelle 18 durch entsprechende Randbedingungen beschrieben wird, also beispielsweise durch einen entsprechenden Durchmesser des modellierten Gefäßsegments und/oder einen resultierenden Strömungswiderstand an der Teilungsstelle 18.

Auf der Grundlage einer fluiddynamischen Simulation 23 oder auf der Grundlage einer Zuordnungsvorschrift 24 kann dann durch die Simulationseinrichtung 21 ein Kennwert 25 ermittelt werden, welcher als der fluiddynamische Kennwert für das Subsegmentmodell 16 verwendet werden kann. Es kann also beispielsweise ein FFR-Wert für das Subsegmentmodell 16 ermittelt werden unter der Annahme, dass sich jenseits der Trennungsstelle 18 der interpolierte Gefäßverlauf 22 anstelle des Subsegmentmodells 17 anschließt. Für die Berechnung des Kennwerts 25 kann zunächst auch ein Gesamtkennwert 26 für das gesamte Segmentmodell 11 durch die Simulationseinrichtung 21 ermittelt werden. Der Gesamtkennwert 26 kann beispielsweise die Gesamtmenge des durch das Gefäßsegment 2 fließenden Fluids, beispielsweise Bluts, angeben. Der Gesamtkennwert 26 kann dann beispielsweise als Randbedingung zum Ermitteln des Kennwerts 25 auf der Grundlage der Geometriedaten des Subsegmentmodells 16 genutzt oder verwendet werden.

Die Analyseeinrichtung 10, die Teilungseinrichtung 13 und die Simulationseinrichtung 21 können z.B. jeweils als ein Programmmodul einer Prozessoreinrichtung CPU der Vorrichtung 1 bereitgestellt sein.

FIG 2 zeigt eine mögliche Darstellung des Segmentmodells 11, wie sie auf der Anzeigeeinrichtung 12 dem Benutzer präsentiert werden kann. Dem Benutzer können mögliche Trennstellen 18, 18' angezeigt werden, mittels eines Cursors 27 kann ein Benutzer zu einem lokalen Querschnitt die Geometriedaten des Segmentmodells 11 auslesen.

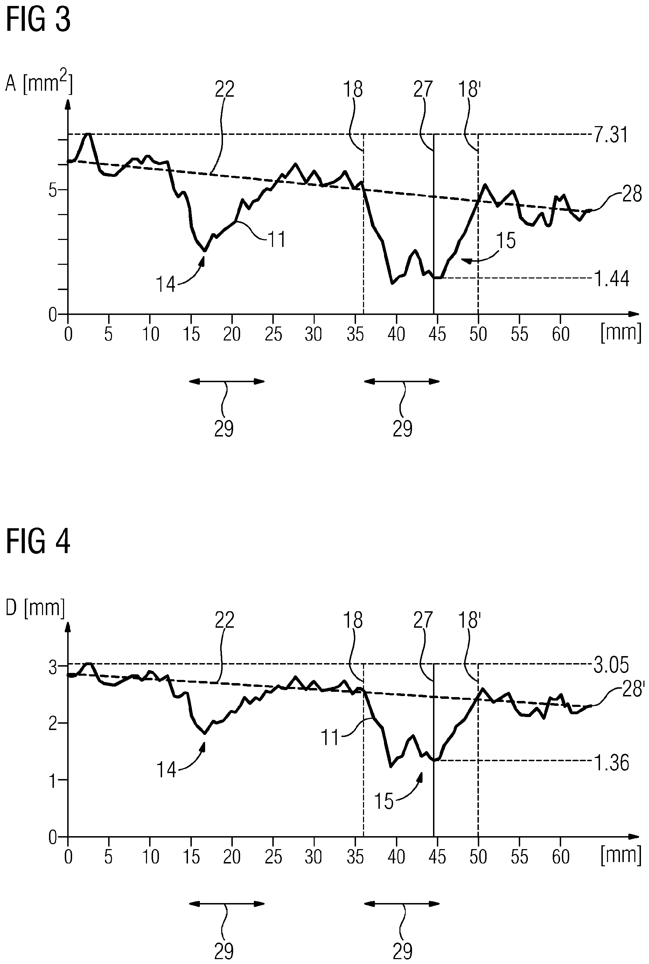

Dies ist in FIG 3 noch einmal veranschaulicht. FIG 3 zeigt eine andere Darstellung des Segmentmodells 11, bei welcher ein ortsabhängiger Verlauf 28 einer Geometrieeigenschaft A des Gefäßsegments 2 nachgestellt oder modelliert ist. Die Geometrieeigenschaft A ist in dem veranschaulichten Beispiel die durchströmbare Querschnittsfläche, die im Bereich der Stenosen 14, 15 kleiner als der virtuelle interpolierte Gefäßverlauf 22 des stenosefreien, das heißt gesunden virtuellen Gefäßes ist. Zum automatisierten Positionieren der Teilungsstellen 18, 18' kann vorgesehen sein, dass der Verlauf 18 kleiner als der Gefäßverlauf 22 sein muss und hierbei eine Mindestlänge 29 der sich ergebenden Stenose 14 ist. Dies kann durch das Teilungskriterium 19 festgelegt sein.

FIG 4 zeigt einen anderen ortsabhängigen Verlauf 28' einer Geometrieeigenschaft D, die beispielsweise den Durchmesser des Gefäßsegments 2 entlang seiner Erstreckungsrichtung darstellt. Auch hier kann zum automatisierten Positionieren der Teilungsstellen 18, 18' das Teilungskriterium 19 besagen, dass die Stenosen 14, 15 eine Geometrieeigenschaft D kleiner als der interpolierte Gefäßverlauf 22 aufweisen muss und die Stenose 14, 15 eine Mindestlänge 29 aufweisen muss.

Die Werte der Geometrieeigenschaft A, D stellen Geometriedaten des Gefäßsegments 2 dar.

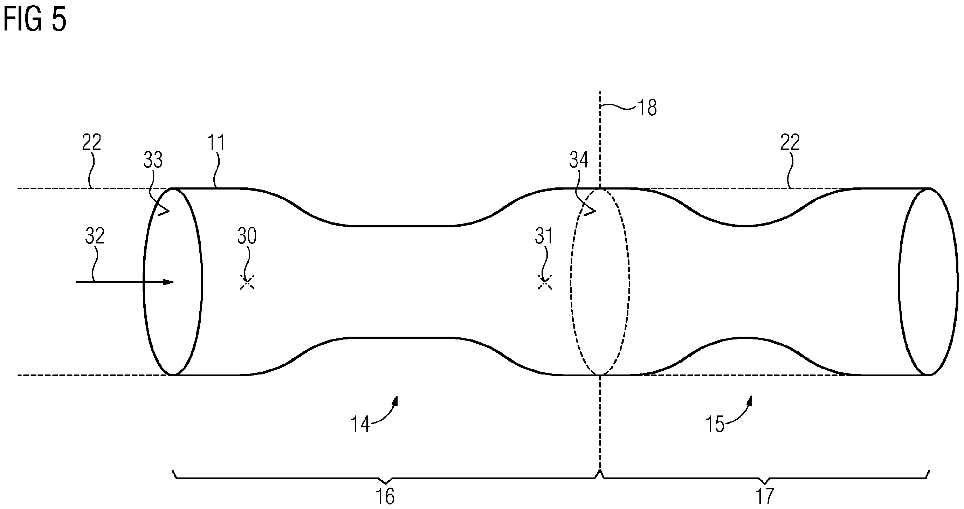

FIG 5 veranschaulicht, wie dann beispielsweise für das Subsegmentmodell 16 die Geometriedaten, beispielsweise die 3D-Daten aus dem 3D-Modell des Segmentmodells 11 und/oder der Verläufe 28, 28' der Geometrieeigenschaften A, D, zugrundegelegt werden können, um für Messpositionen 30, 31 beispielsweise stromaufwärts und stromabwärts der in dem Subsegmentmodell 16 enthaltenen Stenose 14 jeweils mittels der Simulation 23 oder der Zuordnungsvorschrift 24 beispielsweise einen Gefäßdruck oder Fluiddruckwert zu ermitteln, um daraus dann den Kennwert 25 zu ermitteln. Eine Strömungsrichtung 32 ist in FIG 5 durch einen Pfeil veranschaulicht. Stromabwärts der Teilungsstelle 18 wird bei der Ermittlung von Werten an den Messpositionen 30, 31 von dem interpolierten Gefäßverlauf 22 anstelle des verbleibenden Subsegmentmodells 17 ausgegangen. Die Ermittlung der Messwerte 30, 31 kann hierbei mit an sich aus dem Stand der Technik bekannten Methoden, beispielsweise durch die Simulation 23 des Blutflusses, beispielsweise mittels der CFD, oder auf der Grundlage der Zuordnungsvorschrift 24 erzeugt werden. Die Zuordnungsvorschrift 24 kann beispielsweise auf der Grundlage einer Methode des Maschinenlernens bereitgestellt werden.

Somit besteht bei der Vorrichtung 1 die generelle Idee darin, nach Berechnung eines integralen FFR-Wertes, der das gesamte Gefäßsegment 2 mit mehreren Stenosen 14, 15 umfasst, eine weitere diagnostische Analyse für die individuelle Stenose 14, 15 durchzuführen. Dabei wird das Segmentmodell 11 unverändert gelassen, da nur lediglich die einzelnen Einlassflächen 33 und Auslassflächen 34 eines jeweiligen Subsegmentmodells 16, 17 als jeweilige Teilungsstellen 18, 18' festgelegt werden. Dies kann durch den Benutzer oder automatisiert durchgeführt werden. Hierbei muss das Segmentmodell 11 nicht verändert werden, da durch die Teilungsstellen 18 lediglich festgelegt wird, welcher Teil der durch das Segmentmodell 11 repräsentierten Geometriedaten aus dem Segmentmodell 11 genutzt werden soll.

Nachfolgend ist ein beispielhafter Verlauf des durch die Vorrichtung 1 durchgeführten erfindungsgemäßen Verfahrens beschrieben.

Zunächst werden auf Grundlage der Angiographie-Bilddaten 6 der Angiographieaufnahmen Geometrieinformationen über das Gefäßsegment 2 mit mehreren Stenosen 14, 15 gewonnen. Das Segmentmodell 11 kann hierbei ein 3D-Modell sein, aber auch eine Anzahl extrahierter Features oder Merkmale, welche die relevanten Geometrieeigenschaften A, D beschreiben, kann als Segmentmodell 11 verwendet werden.

Mithilfe dieser geometrischen Informationen wird eine erste Kennwertberechnung, beispielsweise eine Berechnung eines FFR-Wertes, durchgeführt, die das gesamte Gefäßsegment 2 umschließt. An dieser Stelle können auch weitere Informationen über den physiologischen Körperzustand miteinfließen.

Es werden durch den Benutzer oder automatisiert Untersegmente oder Subsegmente definiert, die dann jeweils ein Subsegmentmodell 16, 17 festlegen. In jedem Untersegment wird dann eine individuelle Diagnostik für den Kennwert 25, also beispielsweise eine FFR-Diagnostik, berechnet. Dazu werden individuelle Orte, Einlassfläche 33 und Auslassfläche 34 oder mehrere Auslassflächen, definiert, die explizit oder implizit die Stellen der neuen Randbedingungen definieren können.

Es folgt für jedes Subsegment in Form des Subsegmentmodells 16, 17 eine individuelle Berechnung des Kennwerts 25, also beispielsweise eines FFR-Werts, indem diese Berechnung nur die Geometrieeigenschaften A, D aus dem umschriebenen Subsegment verwendet. Das Subsegmentmodell 11 selbst, zum Beispiel die Geometrieinformationen des ordinären Gesamtsegments 2, bleibt unverändert. Eine aufwändige Modellberechnung ist somit nicht notwendig.

Dieser Verfahrensablauf kann sowohl für eine explizite FFR-Berechnung mittels Algorithmen zur Simulation 23 einer Strömung verwendet werden, als auch für implizite Verfahren. Dazu zählen Verfahren, die mittels Maschinenlernen funktionieren. In diesen findet die strömungssimulierte Berechnung des FFR-Werts in einer Trainingsphase statt, während die eigentliche FFR-Berechnung basierend auf den Angiographieaufnahmen gemäß den Bilddaten 6 mittels Maschinenlernen erfolgt, woraus sich die Zuordnungsvorschrift 24 ergibt.

Wie bereits beschrieben kann auch die Unterteilung in Subsegmente automatisch oder manuell erfolgen.

Durch Interpolation des gesunden Gefäßverlaufs 22 an der Einlassfläche 33 und der Auslassfläche 34 wird der Einfluss der jeweils anderen Stenose 15 eliminiert. Die Stenosen 14, 15 lassen sich automatisch unterscheiden, wenn beispielsweise die lokale Fläche A bis auf einen Prozentsatz an dem interpolierten gesunden Gefäßverlauf 22 heranreicht. Ist ein solcher Stenoseabschnitt zu kurz, das heißt beispielsweise weniger als 10 Millimeter, können solche Subsegmente zusammengefasst werden.

Die Ergebnisse können auch Stenose-individuell visualisiert werden, zum Beispiel auf dem beschriebenen Touchscreen.

Der beschriebene Gesamtkennwert 26 stellt auch in vorteilhafter Weise eine Information darüber dar, wie signifikant die Erkrankung des Gefäßsegments 2 insgesamt ist. Die weitere individuelle Bestimmung eines Stenose-individuellen FFR-Werts bietet weitere diagnostische Informationen, die dem Benutzer erlauben, jede Stenose individuell zu beurteilen. Mit der Vorrichtung 1 kann auf der Grundlage des erfindungsgemäßen Verfahrens diese Berechnung schnell durchgeführt werden. Dabei müssen die für die individuelle Berechnung notwendigen Randbedingungen an der Einlassfläche 33 und Auslassfläche 34 jedes Subsegmentmodells 16, 17 angepasst werden, was wesentlich einfacher und schneller ist, als ein vollständiges 3D-Modell zu modifizieren.

Insgesamt zeigt das Beispiel, wie durch die Erfindung ein Verfahren zur Planung einer Behandlung einer Mehrfachstenose bereitgestellt werden kann.

## Patentansprüche

1. Verfahren zum Ermitteln zumindest eines individuellen fluiddynamischen Kennwerts (25) einer Stenose (14, 15) in einem mehrere serielle Stenosen (14, 15) aufweisenden Gefäßsegment (2) eines Körpergefäßes (3), wobei
- aus einer Angiographie-Aufnahmeeinrichtung (5) Angiographie-Bilddaten (6) von dem Gefäßsegment (2) empfangen werden,
- durch eine Analyseeinrichtung (10) auf der Grundlage der Angiographie-Bilddaten (6) Geometriedaten (A, D) des Gefäßsegments (2) ermittelt und zu einem Segmentmodell (11) des Gefäßsegments (2) zusammengefasst werden, - durch eine Teilungseinrichtung (13) in dem Segmentmodell (11) zumindest eine Teilungsstelle (18, 18'), die sich zwischen jeweils zwei der Stenosen (14, 15) befindet, mittels eines vorbestimmten Teilungskriteriums (19) ermittelt und das Segmentmodell (11) an der zumindest einen Teilungsstelle (18, 18') jeweils in Subsegmentmodelle (16, 17) unterteilt wird, und
- durch eine Simulationseinrichtung (21) für zumindest eines der Subsegmentmodelle (16, 17) auf der Grundlage jeweiliger Geometriedaten (A, D) des Subsegmentmodells (16, 17) der jeweilige fluiddynamische Kennwert (25) ermittelt wird,
**dadurch gekennzeichnet, dass**
beim Ermitteln des fluiddynamischen Kennwerts (25) des jeweiligen Subsegmentmodells (16, 17) die jeweiligen Geometriedaten (A, D) jedes übrigen Subsegmentmodells (17, 16) ignoriert werden und stattdessen durch die Simulationseinrichtung (21) zum Ermitteln des fluiddynamischen Kennwerts (25) das Subsegmentmodell (16, 17) an jeder Teilungsstelle (18) durch Interpolation eines gesunden Gefäßverlaufs (22) an einer Einlassfläche (33) und einer Auslassfläche (34) des Subsegmentmodells (16, 17) der Einfluss der jeweils anderen Stenose (15) eliminiert wird, indem das Subsegmentmodell (16, 17) an jeder Teilungsstelle (18) durch Geometriedaten eines virtuellen, stenosefreien interpolierten Gefäßverlaufs (22) ergänzt wird.

2. Verfahren nach Anspruch 1, wobei das Segmentmodell (11) das Gefäßsegment (2) als ein 3D-Modell oder als einen jeweiligen ortsabhängigen Verlauf (28, 28') eines Eigenschaftswerts zumindest einer Geometrieeigenschaft (A, D) des Gefäßsegments (2) beschreibt.

3. Verfahren nach Anspruch 2, wobei die zumindest eine Geometrieeigenschaft (A, D) einen Durchmesser (D) und/oder eine durchströmbare Querschnittsfläche (A) des Gefäßsegments (2) beschreibt.

4. Verfahren nach einem der vorhergehenden Ansprüche, wobei das Segmentmodell (11) zumindest einen Zustandswert eines physiologischen Körperzustands eines das Körpergefäß (3) enthaltenden Körpers (4) umfasst.

5. Verfahren nach einem der vorhergehenden Ansprüche, wobei durch die Teilungseinrichtung (13) die zumindest eine Teilungsstelle (18, 18') ermittelt wird, indem das Teilungskriterium (19) umfasst, dass ein lokaler durchströmbarer Querschnitt (D, A) des Gefäßsegments (2) größer als ein vorbestimmter Prozentsatz des interpolierten, stenosefreien Gefäßverlaufs (2) ist und stromaufwärts und/oder stromabwärts jeweils eine Stenose (14, 15) mit einem lokalen Querschnitts (A, D) kleiner als der Prozentsatz angeordnet ist.

6. Verfahren nach einem der vorhergehenden Ansprüche, wobei einem Benutzer durch eine Anzeigeeinrichtung (12) die Angiographie-Bilddaten (6) und/oder das Segmentmodell (11) angezeigt und als zumindest ein Teil des Teilungskriteriums (19) eine manuelle Teilungsvorgabe (20) von dem Benutzer empfangen wird.

7. Verfahren nach einem der vorhergehenden Ansprüche, wobei eine Teilungsstelle (18, 18') von zwei aneinander angrenzenden Subsegmentmodellen (16, 17) gelöscht wird, falls zumindest eines der beiden Subsegmentmodelle (16, 17) einen Längenabschnitt des Gefäßsegments (2) beschreibt, der kleiner als eine vorbestimmte Mindestlänge (29) ist.

8. Verfahren nach einem der vorhergehenden Ansprüche, wobei durch die Simulationseinrichtung (21) der jeweilige Kennwert (25) des zumindest einen Subsegmentmodells (16, 17) mittels einer Simulation (23) eines Blutflusses in dem Subsegmentmodell (16, 17) ermittelt wird.

9. Verfahren nach einem der vorhergehenden Ansprüche, wobei durch die Simulationseinrichtung (21) der jeweilige Kennwert (25) des zumindest einen Subsegmentmodells (16, 17) mittels einer Zuordnungsvorschrift (24) zum Zuordnen der in dem Subsegmentmodell (16, 17) enthaltenen Geometriedaten (A, D) zu dem Kennwert (25) ermittelt wird.

10. Verfahren nach Anspruch 9, wobei die Zuordnungsvorschrift (24) auf der Grundlage einer Methode des Maschinenlernens aus zumindest einer bekannten Zuordnung von Testgeometriedaten zu einem jeweiligen Kennwert gebildet wird.

11. Verfahren nach einem der vorhergehenden Ansprüche, wobei als der fluiddynamische Kennwert (25) ein Wert einer fraktionellen Flussreserve, FFR, ermittelt wird.

12. Verfahren nach einem der vorhergehenden Ansprüche, wobei durch die Simulationseinrichtung (21) mittels des gesamten Segmentmodells (11) für das gesamte Gefäßsegment (2) ein fluiddynamischer Gesamtkennwert (26) ermittelt wird, welcher den Einfluss aller Stenosen (14, 15) beschreibt, und Sensordaten einer Messeinrichtung, insbesondere Sensordaten eines Druckdrahtes, empfangen werden und mittels der Sensordaten und des Gesamtkennwerts (26) der jeweilige ermittelte Kennwert (25) des zumindest einen Subsegmentmodells (16, 17) kalibriert wird.

13. Vorrichtung (1) zum Ermitteln zumindest eines individuellen fluiddynamischen Kennwerts (25) einer Stenose (14, 15) in einem mehrere serielle Stenosen (14, 15) aufweisenden Gefäßsegment (2) eines Körpergefäßes (3), aufweisend:
- eine Empfangseinrichtung (7), die dazu eingerichtet ist, von einer Angiographie-Aufnahmeeinrichtung (5) Angiographie-Bilddaten (6) von dem Gefäßsegment (2) zu empfangen, und
- eine Prozessoreinrichtung (CPU), die aufweist: eine Analyseeinrichtung (10) zum Ermitteln eines Segmentmodells (11) mit Geometriedaten (A, D) des Gefäßsegments (2) aus den Angiographie-Bilddaten (6), eine Teilungseinrichtung (13) zum Ermitteln zumindest einer Teilungsstelle (18, 18') zwischen jeweils zwei der Stenosen (14, 15) in dem Segmentmodell (11) mittels eines vorbestimmten Teilungskriteriums (19) und zum Einteilen des Segmentmodells (11) in Subsegmentmodelle (16, 17) anhand der zumindest einen Teilungsstelle (18, 18') sowie eine Simulationseinrichtung (21) zum Ermitteln des jeweiligen fluiddynamischen Kennwerts (25) für zumindest eines der Subsegmentmodelle (16, 17) auf der Grundlage der jeweiligen Geometriedaten (A, D) des Subsegmentmodells (16, 17),
**dadurch gekennzeichnet, dass**
die Vorrichtung (1) dazu eingerichtet ist, ein Verfahren nach einem der vorhergehenden Ansprüche durchzuführen.

## Claims

1. Method for ascertaining at least one individual fluid-dynamic characteristic parameter (25) of a stenosis (14, 15) in a vascular segment (2) of a body vessel (3) having a number of serial stenoses (14, 15), wherein
- from an angiography recording device (5) angiography image data (6) of the vascular segment (2) is received,
- geometry data (A, D) of the vascular segment (2) is ascertained by an analysis arrangement (10) on the basis of the angiography image data (6) and combined to form a segment model (11) of the vascular segment (2), - at least one division point (18, 18') which is located between two of the stenoses (14, 15) respectively, is ascertained by a dividing arrangement (13) in the segment model (11) by means of a predetermined division criterion (19) and the segment model (11) is subdivided into subsegment models (16, 17) respectively at the at least one division point (18, 18'), and
- the respective fluid-dynamic characteristic parameter (25) is ascertained by a simulation arrangement (21) for at least one of the subsegment models (16, 17) based on respective geometry data (A, D) of the subsegment model (16, 17),
**characterised in that**
the respective geometry data (A, D) of each remaining subsegment model (17, 16) is ignored when the fluid-dynamic characteristic parameter (25) of the respective subsegment model (16, 17) is ascertained and the influence of the respective other stenosis (15) is instead eliminated by the simulation arrangement (21) for determining the fluid-dynamic characteristic parameter (25) the subsegment model (16, 17) at each division point by interpolation of a heathy vessel path (22) on an inlet surface (33) and an outlet surface (34) of the subsegment model (16, 17), by the subsegment model (16, 17) being supplemented at each division point (18) by geometry data of a virtual stenosis-free interpolated vessel path (22).

2. Method according to claim 1, wherein the segment model (11) describes the vascular segment (2) as a 3D model or as a respective location-dependent path (28, 28') of a property value of at least one geometric property (A, D) of the vascular segment (2).

3. Method according to claim 2, wherein the at least one geometric property (A, D) describes a diameter (D) and/or a cross-sectional surface (A) of the vascular segment (2) through which a flow is possible.

4. Method according to one of the preceding claims, wherein the segment model (11) comprises at least one state value of a physiological physical condition of a body (4) containing the body vessel (3).

5. Method according to one of the preceding claims, wherein the at least one division point (18, 18') is ascertained by the dividing arrangement (13), while the division criterion (19) includes that a local cross section (D, A) of the vascular segment (2) through which a flow is possible is greater than a predetermined percentage of the interpolated, stenosis-free vessel path (2) and upstream and/or downstream there is a respective stenosis (14, 15) with a local cross section (A, D) smaller than the percentage.

6. Method according to one of the preceding claims, wherein the angiography image data (6) and/or the segment model (11) are shown to a user by a display device (12) and a manual division specification (20) is received by the user as at least part of the division criterion (19).

7. Method according to one of the preceding claims, wherein a division point (18, 18') is deleted by two adjacent subsegment models (16, 17) if at least one of the two subsegment models (16, 17) describes a longitudinal section of the vascular segment (2) which is smaller than a predetermined minimum length (29).

8. Method according to one of the preceding claims, wherein the respective characteristic parameter (25) of the at least one subsegment model (16, 17) is ascertained by the simulation arrangement (21) by means of a simulation (23) of the blood flow in the subsegment model (16, 17).

9. Method according to one of the preceding claims, wherein the respective characteristic parameter (25) of the at least one subsegment model (16, 17) is ascertained by the simulation arrangement (21) by means of an assignment rule (24) for assigning the geometry data (A, D) contained in the subsegment model (16, 17) to the characteristic parameter (25).

10. Method according to claim 9, wherein the assignment rule (24) comprises at least one known assignment of test geometry data to a respective characteristic parameter based on a method of machine learning.

11. Method according to one of the preceding claims, wherein a value of a fractional flow reserve, FFR, is ascertained as the fluid-dynamic characteristic parameter (25).

12. Method according to one of the preceding claims, wherein a fluid-dynamic overall parameter (26) which describes the influence of all the stenoses (14, 15) is ascertained for the entire vascular segment (2) by the simulation arrangement (21) by means of the entire segment model (11), and sensor data of a measuring device, in particular, sensor data of a pressure wire, is received and the respective ascertained characteristic parameter (25) of the at least one subsegment model (16, 17) is calibrated by means of the sensor data and the overall parameter (26).

13. Device (1) for ascertaining at least one individual fluid-dynamic characteristic parameter (25) of a stenosis (14, 15) in a vascular segment (2) of a body vessel (3) having a number of serial stenoses (14, 15), having:
- a receiver device (7) which is configured to receive angiography image data (6) of the vascular segment (2) from an angiography recording device (5), and
- a processor device (CPU) which has: an analysis arrangement (10) for ascertaining a segment model (11) with geometry data (A, D) of the vascular segment (2) from the angiography image data (6), a dividing arrangement (13) for ascertaining at least one division point (18, 18') between two of the stenoses (14, 15) in the segment model (11) respectively by means of a predetermined division criterion (19) and for dividing the segment model (11) into subsegment models (16, 17) by means of the at least one division point (18, 18') and a simulation arrangement (21) for ascertaining the respective fluid-dynamic characteristic parameter (25) for at least one of the subsegment models (16, 17) on the basis of the respective geometry data (A, D) of the subsegment model (16, 17),
**characterised in that**
the device (1) is configured to perform a method according to one of the preceding claims.

## Revendications

1. Procédé de détermination d'au moins une valeur (25) caractéristique individuelle de dynamique des fluides d'une sténose (14, 15) dans un segment (2) d'un vaisseau (3) du corps ayant plusieurs sténoses (14, 15) en série, dans lequel
- à partir d'un dispositif (5) d'enregistrement d'angiographie, on reçoit des données (6) d'image d'angiographie du segment (2) du vaisseau,
- par un dispositif (10) d'analyse, sur la base des données (6) d'image d'angiographie, on détermine des données (A, D) de géométrie du segment (2) du vaisseau et on les rassemble en un modèle (11) du segment (2) du vaisseau, - par un dispositif (13) de division du modèle (11) du segment, on détermine, au moyen d'un critère (19) de division déterminé à l'avance, au moins un point (18, 18') de division, qui se trouve entre, respectivement, deux des sténoses (14, 15), et on subdivise le modèle (11) du segment, au au moins un point (18, 18') de division, respectivement, en des sous-modèles (126, 17) de segment, et
- par un dispositif (21) de simulation d'au moins l'un des sous-modèles (16, 17) du segment, on détermine la valeur (25) caractéristique respective de dynamique des fluides sur la base des données (A, D) de géométrie respectives du sous-modèle (16, 17) de segment,
**caractérisé en ce que**
lorsque l'on détermine la valeur (25) caractéristique de dynamique des fluides du sous-modèle (16, 17) de segment respectif, on néglige les données (A, D) respectives de géométrie de chaque autre sous-modèle (17, 16) de segment et, au lieu de celles-ci par le dispositif (21) de simulation, on élimine l'influence de l'autre sténose (15) pour la détermination de la valeur (25) caractéristique de dynamique des fluides du sous-modèle (16, 17) de segment en chaque point (18) de division par interpolation d'un tracé (22) de vaisseau sain à une surface (33) d'entrée et à une surface (34) de sortie du sous-modèle (16, 17) de segment, en complétant le sous-modèle (16, 17) de segment en chaque point (18) de division de données de géométrie d'un tracé (22) de vaisseau virtuel, interpolé sans sténose.

2. Procédé suivant la revendication 1, dans lequel le modèle (11) du segment (2) de vaisseau est un modèle en 3D ou décrit un tracé (28, 28'), qui dépend de l'endroit, d'une valeur d'au moins une propriété (A, D) géométrique du segment (2) de vaisseau.

3. Procédé suivant la revendication 2, dans lequel la au moins une propriété (A, D) géométrique décrit un diamètre (D) et/ou une surface (A) de section transversale de passage du segment (2) du vaisseau.

4. Procédé suivant l'une des revendications précédentes, dans lequel le modèle (11) du segment comprend au moins une valeur d'un état physiologique d'un corps (4) contenant le vaisseau (3).

5. Procédé suivant l'une des revendications précédentes, dans lequel on détermine le au moins un point (18, 18') de division par le dispositif (13) de division, par le fait que le critère (19) de division comprend qu'une section (D, A) transversale locale de passage du segment (2) du vaisseau est plus grande qu'un pourcentage déterminé à l'avancer du tracé (2) du vaisseau interpolé sans sténose et est, en amont et/ou en aval, de, respectivement, une sténose (14, 15) ayant une section (A, D) transversale locale plus petite que le pourcentage.

6. Procédé suivant l'une des revendications précédentes, dans lequel les données (6) d'image d'angiographie et/ou le modèle (11) du segment sont indiqués à un utilisateur par un dispositif (12) d'affichage et il est reçu de l'utilisateur, comme au moins une partie du critère (19) de division, une prescription (20) manuelle de division.

7. Procédé suivant l'une des revendications précédentes, dans lequel on efface un point (18, 18') de division de deux sous-modèles (16, 17) de segment voisins l'un de l'autre, si au moins l'un des deux sous-modèles (16, 17) de segment décrit une longueur de tronçon du segment (2) de vaisseau, qui est plus petite qu'une longueur (29) minimum déterminée à l'avance.

8. Procédé suivant l'une des revendications précédentes, dans lequel on détermine, par le dispositif (21) de simulation, la valeur (25) caractéristique respective du au moins un sous-modèle (16, 17) de segment, au moyen d'une simulation (23) d'un flux sanguin dans le sous-modèle (16, 17) du segment.

9. Procédé suivant l'une des revendications précédentes, dans lequel on détermine, par le dispositif (21) de simulation, la valeur (25) caractéristique respective du au moins un sous-modèle (16, 17) du segment, au moyen d'une prescription (24) d'association pour l'association des données (A, D) de géométrie, contenues dans le sous-modèle (16, 17) du segment, à la valeur (25) caractéristique.

10. Procédé suivant la revendication 9, dans lequel on forme le prescription d'association sur la base d'une méthode d'apprentissage automatique à partir d'au moins une association connue de données de géométrie test à une valeur caractéristique respective.

11. Procédé suivant l'une des revendications précédentes, dans lequel on détermine, comme valeur (25) caractéristique de dynamique des fluides, une valeur de réserve de flux fractionnaire, FFR.

12. Procédé suivant l'une des revendications précédentes, dans lequel on détermine, par le dispositif (21) de simulation, au moyen de tout le modèle (11) de segment pour tout le segment (2) du vaisseau, une valeur (26) caractéristique d'ensemble de dynamique des fluides, qui décrit l'influence de toutes les sténoses (14, 15), et on reçoit des données de capteur d'un dispositif de mesure, notamment des données de capteur d'un fil manométrique et, au moyen des données de capteur et de la valeur (26) caractéristique d'ensemble, on étalonne la valeur (25) caractéristique déterminée, respectivement, du au moins un sous-modèle (16, 17) du segment.

13. Installation (1) de détermination d'au moins une valeur (25) caractéristique individuelle de dynamique des fluides d'une sténose (14, 15) dans un segment (2) d'un vaisseau (3) du corps ayant plusieurs sténoses (14, 15) en série, comportant :
- un dispositif (7) de réception, conçu pour recevoir d'un dispositif (5) d'enregistrement d'angiographie des données (6) d'image d'angiographie du segment (2) du vaisseau, et
- un dispositif (CPU) à processeur, qui a : un dispositif (10) d'analyse pour déterminer un modèle (11) de segment, ayant des données (A, D) de géométrie du segment (2) du vaisseau, à partir des données (6) d'image d'angiographie, un dispositif (13) de division pour déterminer au moins un point (18, 18') de division entre, respectivement, deux des sténoses (14, 15) dans le modèle (11) du segment, au moyen d'un critère (19) de division déterminé à l'avance et pour diviser le modèle (11) du segment en sous-modèles (16, 17) du segment à l'aide du au moins un point (18, 18') de division, ainsi qu'un dispositif (21) de simulation pour déterminer la valeur (25) caractéristique de dynamique des fluides d'au moins l'un des sous-modèles (16, 17) du segment sur la base des données (A, D) de géométrie respectives du sous-modèle (16, 17) du segment,
**caractérisée en ce que**
l'installation est conçue pour effectuer un procédé suivant l'une des revendications précédentes.
